# EUROPEAN PATENT SPECIFICATION

(11) **EP 4 092 135 B1**
(45) Date of publication and mention of the grant of the patent: **09.04.2025**
(21) Application number: 20914690.1
(22) Date of filing: 17.01.2020
(51) Int. Cl.: C12Q 1/6869, C12Q 1/6806

(54) **METHOD FOR SYNCHRONOUSLY SEQUENCING SENSE STRAND AND ANTISENSE STRAND OF DNA**
VERFAHREN ZUR SYNCHRONEN SEQUENZIERUNG DES SENSE-STRANGS UND DES ANTISENSE-STRANGS VON DNA
PROCÉDÉ DE SÉQUENÇAGE SYNCHRONE DE BRIN SENS ET DE BRIN ANTISENS D'ADN

(43) Date of publication of application: 23.11.2022
(73) Proprietor: MGI Tech Co., Ltd., Shenzhen, Guangdong 518083 (CN)
(72) Inventor: GONG, Meihua, Shenzhen, Guangdong 518083 (CN); LIU, Shengmao, Shenzhen, Guangdong 518083 (CN); XU, Chongjun, Shenzhen, Guangdong 518083 (CN); ZHOU, Shuang, Shenzhen, Guangdong 518083 (CN); WANG, Jingjing, Shenzhen, Guangdong 518083 (CN); LI, Jiguang, Shenzhen, Guangdong 518083 (CN); WEI, Xiaofang, Shenzhen, Guangdong 518083 (CN); JIANG, Hui, Shenzhen, Guangdong 518083 (CN); LIU, Jian, Shenzhen, Guangdong 518083 (CN)
(74) Representative: Dehns Germany Partnerschaft mbB
(86) International application number: PCT/CN2020/072727
(87) International publication number: WO 2021/142769

(56) References cited:
- WO-A1-2019/086531
- WO-A1-2019/209946
- WO-A2-2019/028470
- CN-A- 103 602 735
- CN-A- 104 379 766

## Description

### TECHNICAL FIELD

The present application relates to the technical field of nucleic acid sequencing, and particularly, to a method for synchronously sequencing a sense strand and an antisense strand of insert DNA.

### BACKGROUND

Currently, high-throughput sequencing methods mainly include single-end sequencing and paired-end/mate-paired (PE/MP) sequencing. PE/MP sequencing is also referred to as bi-directional sequencing for determining sequences at the two ends of a long DNA fragment, and the sequences at the two ends form a "pair". A distance between the sequences at the two ends is a length of the insert DNA fragment for sequence assembly, alignment, etc. For duplication, deletion and insertion of genome, this method is more accurate, and has a wider coverage on the genome. The difference between the paired-end sequencing and the mate-paired sequencing lies in the way of constructing the library. At present, the paired-end sequencing is dominant, as it increases the length of sequencing and can also provide a new method for structural variation analysis.

With respect to the PE/MP sequencing method, different sequencing platforms currently have different sequencing solutions, but they are generally required to sequence one strand read1 of DNA first, and thereafter sequence the complementary strand read2. For example, the PE/MP sequencing developed by Illumina, which takes the highest market share, mainly employees bridge amplification. Specifically, DNA fragmentation is first performed, adapters containing sequencing primer binding sites are added at both ends, and a template strand for a first-round sequencing of read1 is removed after the first round of sequencing is finished. Then, the complementary strand is in-situ regenerated and amplified under guidance by a Paired-End Module, so as to reach the number of templates to be used in the second round of sequencing, and the second round of sequencing by synthesis is performed on the complementary strand read2. WO2019/209946 A1 (QIAGEN SCIENCES LLC [US]) discloses that a duplex target is circularized followed by sequential sequencing of both template strands present in rolling circle amplification (RCA) products. WO2019/086531 A1 (HOFFMANN LA ROCHE [CH]; ROCHE DIAGNOSTICS GMBH [DE] ET AL.) discloses RCA followed by multiple displacement amplification (MDA) to generate sequencing templates.

The existing PE/MP sequencing has the problems of lower sequencing throughput and higher sequencing cost, as the two strands of DNA need to be sequenced one after another.

### SUMMARY

The present disclosure provides a method for synchronously sequencing a sense strand and an antisense strand of insert DNA, capable of simultaneously sequencing the insert DNA from both ends of the insert DNA, and synchronously sequencing the sense and antisense strands of the insert DNA. That is, one stand read1 and a complementary strand read2 can be simultaneously sequenced, thereby greatly saving the time and costs for sequencing, and increasing the sequencing throughput.

The above are achieved by the present disclosure through the following technical solutions.

A method for synchronously sequencing a sense strand and an antisense strand of insert DNA, includes: performing a first round of rolling circle amplification using a circular DNA molecule to be sequenced as an amplification template, to generate a first partial template strand; hybridizing the first partial template strand with blocked or unblocked amplification primers, and performing a second round of rolling circle amplification using the circular DNA molecule to be sequenced as an amplification template to generate a second partial template strand; selecting any one partial template strand of the first partial template strand or the second partial template strand as a read1 strand sequencing template, and generating a read2 strand sequencing template by performing multiple displacement amplification using the other partial template strand as an amplification template; and hybridizing the read1 strand sequencing template with read1 strand sequencing primers, hybridizing the read2 strand sequencing template with read2 strand sequencing primers, and simultaneously performing read1 strand sequencing and read2 strand sequencing to obtain sequences of the sense strand and the antisense strand of the insert DNA.

In preferable embodiments, the blocked amplification primers are 3'-end phosphorylated read1 strand sequencing primer; and the unblocked amplification primers are multiple-displacement-amplification primers.

In preferable embodiments, the above-described method includes the following steps: performing the first round of rolling circle amplification using a single-stranded circular DNA molecule of the DNA as the amplification template, to generate a DNA nano ball; hybridizing the first partial template strand generated by the first round of rolling circle amplification with 3'-end phosphorylated read1 strand sequencing primers; performing the second round of rolling circle amplification on the DNA nano ball to extend a 3'-end of the template strand to generate the second partial template strand, the second partial template strand being used as the read1 strand sequencing template; dephosphorylating the 3'-end phosphorylated read1 strand sequencing primers, generating the read2 strand sequencing template by performing the multiple displacement amplification, and hybridizing the read2 strand sequencing template with the read2 strand sequencing primers; hybridizing the read1 strand sequencing template with the read1 strand sequencing primers to obtain a DNA nano ball template ready to be sequenced, the DNA nano ball template being hybridized with the read1 strand sequencing primers and the read2 strand sequencing primers; and simultaneously performing the read1 strand sequencing and the read2 strand sequencing using the read1 strand sequencing primers and the read2 strand sequencing primers, to obtain the sequences of the sense strand and the antisense strand of the insert DNA.

In preferable embodiments, the above-described method includes the following steps: performing the first round of rolling circle amplification using a single-stranded circular DNA molecule as the amplification template, to generate a DNA nano ball; hybridizing the first partial template strand generated by the first round of rolling circle amplification with multiple-displacement-amplification primers; performing the second round of rolling circle amplification on the DNA nano ball to extend a 3'-end of the template strand to generate the second partial template strand, the second partial template strand being used as the read1 strand sequencing template; generating the read2 strand sequencing template by performing the multiple displacement amplification using the multiple-displacement-amplification primers, and hybridizing the read2 strand sequencing template with the read2 strand sequencing primers; hybridizing the read1 strand sequencing template with the read1 strand sequencing primers to obtain a DNA nano ball template ready to be sequenced, the DNA nano ball template being hybridized with the read1 strand sequencing primers and the read2 strand sequencing primers; and simultaneously performing the read1 strand sequencing and the read2 strand sequencing using the read1 strand sequencing primers and the read2 strand sequencing primers, to obtain the sequences of the sense strand and the antisense strand of insert DNA.

In preferable embodiments, the above-described method includes the following steps: performing the first round of rolling circle amplification using a single-stranded circular DNA molecule of the insert DNA as the amplification template, to generate a DNA nano ball; hybridizing the first partial template strand generated by the first round of rolling circle amplification with 3'-end phosphorylated read1 strand sequencing primers; performing the second round of rolling circle amplification on the insert DNA nano ball to extend a 3'-end of the template strand, wherein in the second round of rolling circle amplification, a dUTP-containing mix is used first to extend the template strand to obtain a template strand having a part containing U bases, and a dNTP mix is used then to further extend the template strand to generate the second partial template strand; hybridizing the second partial template strand with multiple-displacement-amplification primers, and performing the multiple displacement amplification to generate the read2 strand sequencing template; hybridizing the read2 strand sequencing template with the read2 strand sequencing primers, and dephosphorylating the 3'-end phosphorylated read1 strand sequencing primers to obtain a DNA nano ball template ready to be sequenced, the DNA nano ball template being hybridized with the read1 strand sequencing primers and the read2 strand sequencing primers; and simultaneously performing the read1 strand sequencing and the read2 strand sequencing using the read1 strand sequencing primers and the read2 strand sequencing primers, to obtain the sequences of the sense strand and the antisense strand of the insert DNA.

In preferable embodiments, in the above-described method, a copy number of the read1 strand sequencing template and a copy number of the read2 strand sequencing template are controlled by controlling time of the rolling circle amplification and time of the multiple displacement amplification.

In preferable embodiments, the copy number of the read1 strand sequencing template and the copy number of the read2 strand sequencing template have a difference in folds, and sequencing bases are positioned by using a signal difference caused by the difference in folds during the sequencing.

In preferable embodiments, in the above-described method, different specific antibodies with different fluorescence are added during the sequencing to specifically recognize different bases with blocking groups to acquire base signals.

In preferable embodiments, the above-described sequencing includes: firstly adding the bases with blocking groups, adding specific antibodies with different fluorescence that are capable of specifically recognizing bases with blocking groups and acquiring signals, eluting the antibodies to remove the signals, and subsequently removing the blocking groups, to enter a next round of cycle. In preferable embodiments, the above-described sequencing is performed on an MGISEQ-2000 sequencer.

In the sequencing method of the present disclosure, through two rounds of rolling circle amplification and the multiple displacement amplification, a DNA nano ball template ready to be sequenced can be obtained, and since the DNA nano ball template is hybridized with the read1 strand sequencing primers and the read2 strand sequencing primers, the read1 strand sequencing and the read2 strand sequencing can be performed simultaneously to obtain the sequences of the sense strand and the antisense strand of insert DNA. In this way, the sequencing time and sequencing cost are greatly saved, and the sequencing throughput is improved.

### BRIEF DESCRIPTION OF DRAWINGS

FIG. 1 is a principle schematic diagram of a first scheme of a method for synchronously sequencing a sense strand and an antisense strand of an insert DNA according to embodiments of the present disclosure;
FIG. 2 is a principle schematic diagram of a second scheme of a method for synchronously sequencing a sense strand and an antisense strand of an insert DNA according to embodiments of the present disclosure;
FIG. 3 is a principle schematic diagram of a third scheme of a method for synchronously sequencing a sense strand and an antisense strand of an insert DNA according to embodiments of the present disclosure;
FIG. 4 is a graph illustrating sequencing signal results of a first scheme of the method for synchronously sequencing a sense strand and an antisense strand of an insert DNA according to embodiments of the present disclosure;
FIG. 5 is a graph illustrating sequencing signal results of a second scheme of the method for synchronously sequencing a sense strand and an antisense strand of an insert DNA according to embodiments of the present disclosure; and
FIG. 6 is a graph illustrating sequencing signal results of a third scheme of the method for synchronously sequencing a sense strand and an antisense strand of an insert DNA according to embodiments of the present disclosure.

### DESCRIPTION OF EMBODIMENTS

Hereinafter, the present disclosure is described in detail by means of specific embodiments in conjunction with the accompanying drawings. In the following embodiments, many detailed descriptions are provided to facilitate the understanding of the present disclosure. However, those skilled in the art can readily realize that some of these features may be omitted under different circumstances, or may be replaced by other materials or methods.

In a method of the present disclosure, through two rounds of rolling circle amplification and the multiple displacement amplification, a DNA nano ball template ready to be sequenced can be obtained. The template for a read2 strand or the template for a read1 strand can be generated by controlling the time of the rolling circle amplification (RCA) and the time of the multiple displacement amplification (MDA). That is, the copy number of the read2 strand and the copy number of the read1 strand are both controllable. In some schemes, a template containing both the read2 stands and the read1 strands is generated and hybridized with the read2 strand sequencing primers and the read1 strand sequencing primers, and the sequencing bases can be positioned for the read1 strand and the read2 strand through a signal difference caused by a difference between the copy number of the read2 strand and the copy number of the read1 strand, for example, the signal of the read2 being 2 times or other times a signal of the read1, to simultaneously sequence the read1 strand and the read2 strand, thereby synchronously sequencing the sense and antisense strands of the insert DNA.

Specifically, in some embodiments of the present disclosure, a method for synchronously sequencing a sense strand and an antisense strand of an insert DNA includes the following steps: performing a first round of rolling circle amplification using a circular DNA molecule to be sequenced as an amplification template, to generate a first partial template strand; hybridizing the first partial template strand with blocked or unblocked amplification primers, and performing a second round of rolling circle amplification using the circular DNA molecule to be sequenced as an amplification template, to generate a second partial template strand; selecting one of the first partial template strand or the second partial template strand as a read1 strand sequencing template, and generating a read2 strand sequencing template by performing multiple displacement amplification using the other partial template strand as an amplification template; and hybridizing the read1 strand sequencing template with read1 strand sequencing primers, hybridizing the read2 strand sequencing template with read2 strand sequencing primers, and simultaneously performing read1 strand sequencing and read2 strand sequencing to obtain sequences of the sense strand and the antisense strand of the insert DNA.

In the present disclosure, the rolling circle amplification includes two stages, i.e., the first round of rolling circle amplification and the second round of rolling circle amplification, which generate the first partial template strand and the second partial template strand respectively. By controlling the time of the rolling circle amplification, a length of the first partial template strand and a length of the second partial template strand can be controlled, and accordingly, a copy number of the read1 strand and a copy number of the read2 strand can be controlled. The rolling circle amplification is generally carried out under the action of DNA polymerase, and the most common DNA polymerase is phi29 DNA polymerase.

In the present disclosure, either one of the first partial template strand and the second partial template strand can serve as the read1 strand sequencing template, and the other of the first partial template strand and the second partial template strand can be used as the template strand of the multiple displacement amplification to generate the read2 strand sequencing template.

In the present disclosure, subsequent to the first round of rolling circle amplification and prior to the second round of rolling circle amplification, the first partial template strand is hybridized with the blocked or unblocked amplification primers. In this way, on the one hand, the first partial template strand can be temporarily blocked, and on the other hand, these amplification primers can be used as primer sequences for the subsequent multiple displacement amplification, or as primers for the subsequent read1 strand sequencing.

In the present disclosure, these amplification primers are blocked or unblocked. The blocked amplification primer generally refers to a primer whose 3'-end is blocked by a blocking group and cannot be extended under the action of a polymerase. In an embodiment of the present disclosure, the blocked amplification primer is a 3'-end phosphorylated read1 strand sequencing primer. Since the 3'-end of the read1 strand sequencing primer is phosphorylated and cannot be extended, it remains unchanged during the second round of rolling circle amplification. Subsequent to the second round of rolling circle amplification, the 3'-end phosphorylated read1 strand sequencing primer can be dephosphorylated such that the 3'-end group of the read1 strand sequencing primer is changed into hydroxyl and thus the 3'-end can be extended under the action of a polymerase. Accordingly, the dephosphorylated read1 strand sequencing primer can be used as a primer for the subsequent multiple displacement amplification or a primer for the subsequent read1 strand sequencing, and the specific use thereof can be determined according to the design form of the technical solutions.

When the unblocked amplification primer is bound to the first partial template strand, and during the second round of rolling circle amplification, this amplification primer will initiate an extension reaction, without affecting the extension reaction of the rolling circle amplification. After the second round of rolling circle amplification, the MDA primer extension reaction can be performed under suitable reaction conditions to generate the read2 strand sequencing template. Thus, in an embodiment of the present disclosure, the unblocked amplification primer is a multiple-displacement-amplification primer.

The method for synchronously sequencing a sense strand and an antisense strand of an insert DNA according to the present disclosure can be implemented by means of various specific technical solutions. The present disclosure provides three typical detailed solutions, but the technical solutions are not limited thereto. It should be understood that on the basis of these three technical solutions, those skilled in the art can also make other suitable modified solutions.

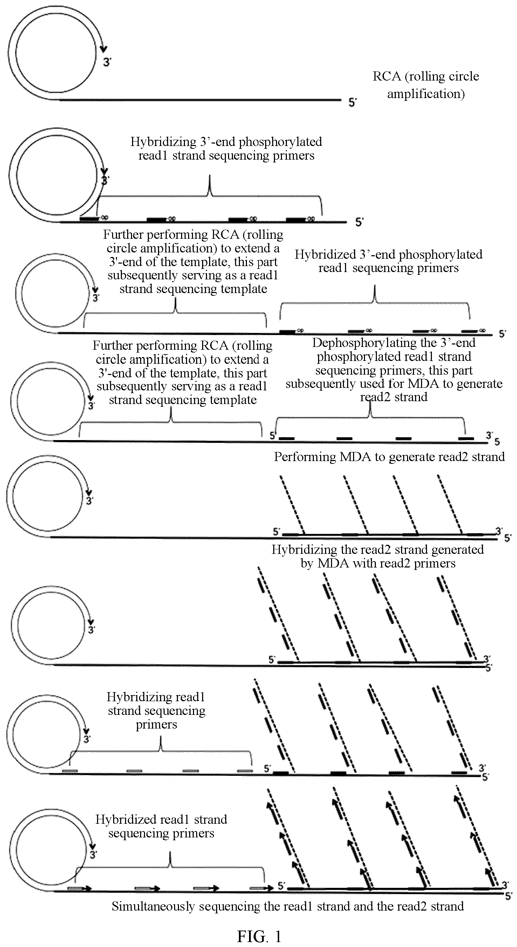

As illustrated in FIG. 1, a first detailed technical scheme of the method for synchronously sequencing a sense strand and an antisense strand of an insert DNA includes: performing a first round of rolling circle amplification (RCA) using a single-stranded circular DNA molecule of the insert DNA to be sequenced as the amplification template, to generate a DNA nano ball (DNB), without adding a stop solution; hybridizing 3'-end phosphorylated read1 strand sequencing primers on the first partial template strand generated by the first round of rolling circle amplification; then performing a second round of rolling circle amplification on the DNB to extend a 3'-end of the template strand, the extended template strand subsequently serving as a sequencing template of a read1 strand; then dephosphorylating the hybridized 3'-end phosphorylated read1 strand sequencing primers to perform multiple displacement amplification (MDA) to generate the read2 strand sequencing template, and hybridizing the read2 strand sequencing template with read2 strand sequencing primers; finally hybridizing the read1 strand sequencing primers, so that the DNA nano ball template includes the hybridized read1 strand sequencing primers as well as the read2 strand sequencing template generated and hybridized with read2 strand sequencing primers; and simultaneously performing read1 strand sequencing and read2 strand sequencing using the read1 strand sequencing primers and the read2 strand sequencing primers, to obtain the sequences of the sense strand and the antisense strand of the insert DNA. Since the time of the rolling circle amplification (RCA) and the time of the multiple displacement amplification (MDA) to generate the read2 strands are controllable, the copy number of the read2 strand and the copy number of the read1 strand can be controlled. The sequencing bases can be positioned for the read1 strand and the read2 strand simply by a signal difference brought by the copy number of the read2 strand and the copy number of the read1 strand, for example, the signal of the read2 being 2 times or other times the signal of the read1.

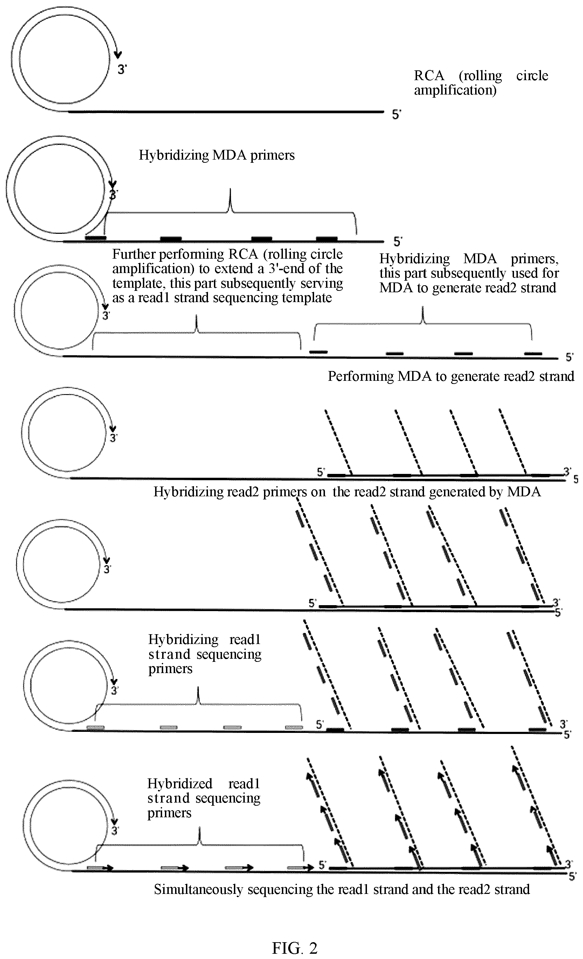

As illustrated in FIG. 2, a second detailed technical scheme of the method for synchronously sequencing a sense strand and an antisense strand of an insert DNA includes: performing a first round of rolling circle amplification (RCA) using a single-stranded circular DNA molecule as the amplification template, to generate a DNA nano ball (DNB), without adding a stop solution; hybridizing multiple-displacement-amplification (MDA) primers; then performing a second round of rolling circle amplification on the DNB to extend a 3'-end of the template strand, the extended template strand subsequently serving as a sequencing template of the read1 strand, performing multiple displacement amplification (MDA) on the part hybridized with the MDA primers, to generate the read2 strand sequencing template, and hybridizing the read2 strand sequencing template with the read2 strand sequencing primers; immediately after that, hybridizing the read1 strand sequencing template with the read1 strand sequencing primers, so that the DNB template includes the hybridized read1 strand sequencing primers as well as the read2 strand sequencing template generated and hybridized with the read2 strand sequencing primers; and finally, simultaneously performing read1 strand sequencing and read2 strand sequencing using the read1 strand sequencing primers and the read2 strand sequencing primers, to obtain the sequences of the sense strand and the antisense strand of the insert DNA. Since the time of the rolling circle amplification (RCA) and the time of the multiple displacement amplification (MDA) to generate the read2 strand template are controllable, the copy number of the read2 strand and the copy number of the read1 strand can be controlled. The sequencing bases can be positioned for the read1 strand and the read2 strand simply by a signal difference brought by the copy number of the read2 strand and the copy number of the read1 strand, for example, the signal of the read2 being 2 times or other times the signal of the read1.

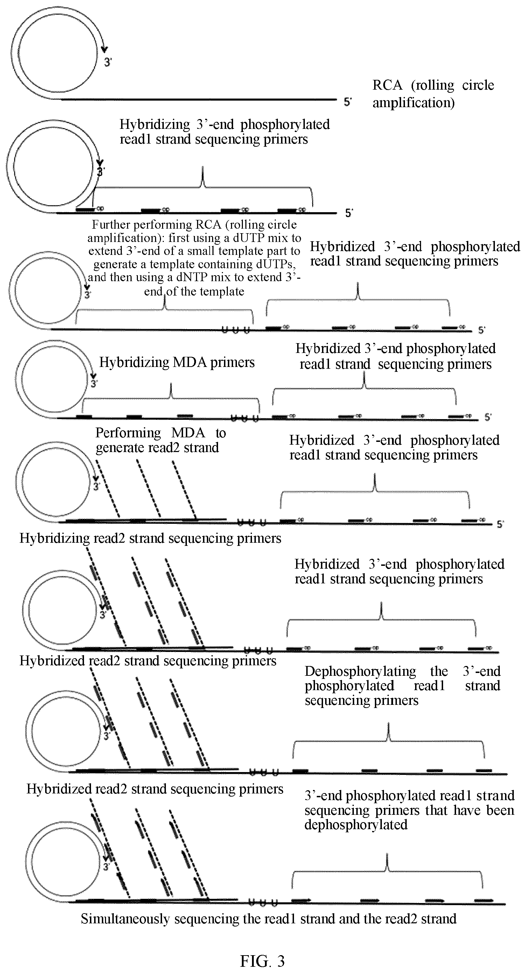

As illustrated in FIG. 3, a third detailed technical scheme of the method for synchronously sequencing a sense strand and an antisense strand of an insert DNA includes: performing a first round of rolling circle amplification (RCA) using a single-stranded circular DNA molecule as the amplification template, to generate a DNA nano ball (DNB), without adding a stop solution; hybridizing the first partial template strand generated by the first round of rolling circle amplification with 3'-end phosphorylated read1 strand sequencing primers, which can be subsequently dephosphorylated for read1 strand sequencing; then performing the second round of rolling circle amplification on the DNB in two stages to extend a 3'-end of the template strand, in which a dUTP-containing mix is used to extend the 3'-end of the template strand to obtain a template strand having a part containing U bases, the part of the template strand containing U bases cannot be recognized by phi29 DNA polymerase and thus cannot be amplified by MDA, and a dNTP mix is then used for normal extension to generate the second partial template strand which is used subsequently for MDA amplification; hybridizing the second partial template strand with the read1 strand sequencing primers, performing multiple displacement amplification (MDA) to generate the read2 strand sequencing template, hybridizing the read2 strand sequencing template with the read2 strand sequencing primers, and then dephosphorylating the 3'-end phosphorylated read1 strand sequencing primers, so that the DNB template includes the hybridized read1 strand sequencing primers as well as the read2 strand sequencing template generated and hybridized with the read2 strand sequencing primers; and finally, simultaneously performing read1 strand sequencing and read2 strand sequencing using the read1 strand sequencing primers and the read2 strand sequencing primers, to obtain the sequences of the sense strand and the antisense strand of the insert DNA. Since the time of the rolling circle amplification (RCA) and the time of the multiple displacement amplification (MDA) to generate the read2 strand are controllable, the copy number of the read2 strand and the copy number of the read1 strand can be controlled. The sequencing bases can be positioned for the read1 strand and the read2 strand simply by a signal difference brought by the copy number of the read2 strand and the copy number of the read1 strand, for example, the signal of the read2 being 2 times or other times the signal of the read1.

The methods according to the present disclosure, through appropriate modification and variation, are also applicable to synchronous sequencing of a sense strand and an antisense strand of other double-stranded DNAs or DNAs of other structures. In the variant technical solutions, different means are required to realize a method for synchronously sequence DNA sense strand and antisense strand in which both the read1 strand sequencing template and the read2 strand sequencing template are formed, and there is a certain difference between the copy number of the read2 strand and the copy number of the read1 strand so as to form a difference in terms of signal intensity between the read1 and the read2 for distinguishing and positioning the two templates.

In the method of the present disclosure, during the sequencing, different specific antibodies with different fluorescence can be added to specifically identify the different bases carrying blocking groups, so as to collect base signals. For example, in one embodiment, the bases carrying blocking groups are added firstly, then the specific antibodies with different fluorescence, which can specifically recognize the A and T bases carrying blocking groups, are added, signals are acquired, the antibodies are then eluted to remove signals, specific antibodies with different fluorescence, which can specifically recognize the C and G bases carrying blocking groups, are then added, and signals are acquired. In this way, in each round of reaction, only two types of fluorescent signals are emitted, and the read2 and the read1 have a signal difference, so as to achieve a better signal identification effect, thereby improving the sequencing quality of synchronous sequencing.

The technical solutions and effects of the present disclosure will be described in details below by way of examples. It should be understood that these examples are merely illustrative and should not be construed as limiting the present disclosure.

### Example 1

### 1. Instruments

MGISEQ-2000 sequencer, MGISEQ-2000 sequencing flow cell (715nm), Mini loader, PCR machine, PCR 8-connected tubes, a set of pipettors, and High-speed centrifuge.

### 2. Reagents

Reagents used in the present example are listed in the following Table 1.

**Table 1**

| Reagent name | Manufacturer |
|---|---|
| DNA nano ball preparation buffer | Sigma-Aldrich |
| 3'-end phosphorylated read1 strand sequencing primers | MGI |
| PNK enzyme (polynucleotide kinase) | MGI |
| Read1 strand sequencing primers | MGI |
| Read2 strand sequencing primers | MGI |
| MDA primers | MGI |
| MGISEQ-2000RS high-throughput sequencing reagents | MGI |
| DNA nano ball preparation enzyme mix I | MGI |
| DNA nano ball preparation enzyme mix I (dUTP-containing mix) | MGI |
| DNA nano ball preparation enzyme mix II | MGI |
| 1XTE buffer | MGI |
| 5XSSC buffer | MGI |
| Sequencing flow cell | MGI |
| MGISEQ-2000RS high-throughput sequencer | MGI |
| DNA nano ball loading buffer I | MGI |
| DNA nano ball loading buffer II | MGI |
| *Escherichia coli* library | MGI |

### 3. Reagent preparation:

1) Dissolution of 3'-end phosphorylated read1 strand sequencing primers:
A 1.5 ml centrifuge tube containing the powder of the 3'-end phosphorylated read1 strand sequencing primers was placed and centrifuged on a high-speed centrifuge (Eppendorf, 5415D) for 5 minutes at a maximum speed. The primers were dissolved by 1XTE buffer to a 100 µM site-occupying primer mother solution;
2) 1 µM working solution of the 3'-end phosphorylated read1 strand sequencing primers was prepared according to Table 2 below:

**Table 2**

| Reagent name | Volume |
|---|---|
| 100µM mother solution of 3'-end phosphorylated read1 strand sequencing primers | 100 µl |
| 5XSSC buffer | 9.9 ml |
| Total | 10 ml |

3) PNK enzyme reagent (for dephosphorylation) was prepared according to Table 3 below:

**Table 3**

| Reagent name | Final concentration |
|---|---|
| 10U T4PNK (BGI) | 0.1U |
| 10X reaction buffer (PH5.9) | 1X |

### 4. Operation steps:

1) With reference to "Instructions for MGISEQ-2000RS high-throughput sequencing reagent kit", the preparation of DNA nano balls was performed on *Escherichia coli* library, 3 pieces of sequencing flow cells were prepared, and DNA nano balls were loaded on the MGISEQ-2000 sequencing flow cells (715 nm); and then according to each of the three technical solutions as described in the present disclosure, the read2 template and the read1 template were generated.
2) Three sequencing kits were prepared in accordance with the "Instructions for MGISEQ-2000RS high-throughput sequencing reagent kit".
3) According to the "Instructions for MGISEQ-2000RS high-throughput sequencing reagent kit", the sequencing kits and the chips were placed on MGI2000-RS sequencer, a corresponding script was selected, followed by setting SE1 and carrying out the sequencing. In order to prove the feasibility of this scheme, in this sequencing, the read2 strand sequencing primers were hybridized first, and a first cycle (cycle1) of sequencing of read2 strand was performed. After the sequencing was completed, the read2 sequencing strand was blocked, and then the read1 strand sequencing primers were hybridized to perform a first cycle (cycle1) of sequencing of read1 strand.

### 5. Results:

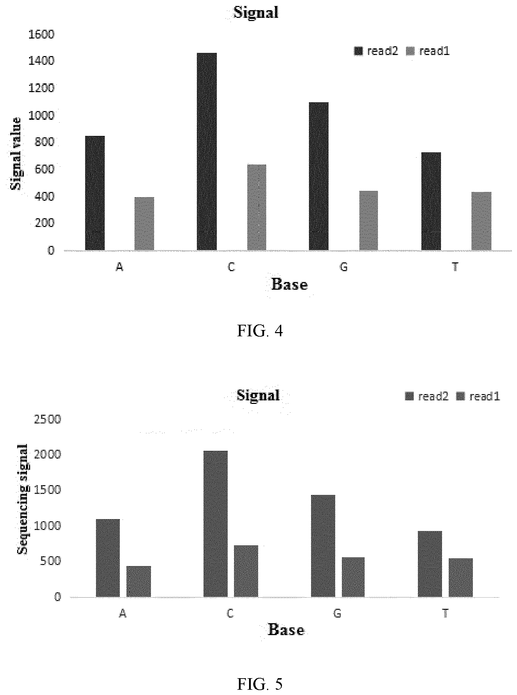

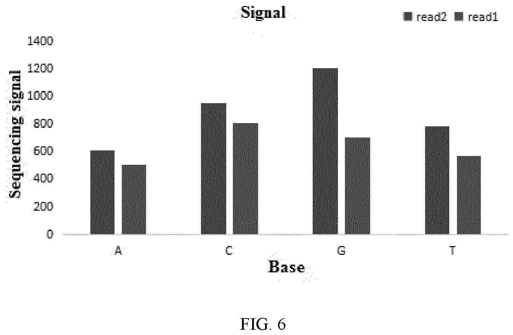

The results are shown in FIG. 4 to FIG 6. FIG. 4 shows the sequencing signal results of the first scheme of the present disclosure, FIG. 5 shows the sequencing signal results of the second scheme of the present disclosure, and FIG. 6 shows the sequencing signal results of the third scheme of the present disclosure. The results indicate that above three schemes are all feasible, but the first scheme and the second scheme are better than the third scheme. Specifically, the signal of read2 was about 2 times the signal of read1 in the first scheme, the signal of read2 was about 2.5 times the signal of read1 in the second scheme, while the signal of read2 was about 1.2 times the signal of read1 in the third scheme.

The specific examples as described above are intended to explain the present disclosure, only for helping to understand the present disclosure, rather than limiting the present disclosure. Those skilled in the art to which the present disclosure pertains can made several simple deductions, modifications or substitutions based on the idea of the present disclosure.

## Claims

1. A method for synchronously sequencing a sense strand and an antisense strand of an insert DNA, comprising:
performing a first round of rolling circle amplification using a circular DNA molecule of the insert DNA to be sequenced as an amplification template, to generate a first partial template strand;
hybridizing the first partial template strand with blocked or unblocked amplification primers, and performing a second round of rolling circle amplification using the circular DNA molecule as an amplification template to generate a second partial template strand;
selecting any one partial template strand of the first partial template strand or the second partial template strand as a read1 strand sequencing template, and generating a read2 strand sequencing template by performing multiple displacement amplification using the other partial template strand as an amplification template; and
hybridizing the read1 strand sequencing template with read1 strand sequencing primers, hybridizing the read2 strand sequencing template with read2 strand sequencing primers, and simultaneously performing read1 strand sequencing and read2 strand sequencing to obtain sequences of the sense strand and the antisense strand of the insert DNA.

2. The method according to claim 1, wherein the blocked amplification primers are 3'-end phosphorylated read1 strand sequencing primers; and the unblocked amplification primers are multiple-displacement-amplification primers.

3. The method according to claim 1, comprising:
performing the first round of rolling circle amplification using a single-stranded circular DNA molecule of the insert DNA as the amplification template, to generate a DNA nano ball;
hybridizing the first partial template strand generated by the first round of rolling circle amplification with 3'-end phosphorylated read1 strand sequencing primers;
performing the second round of rolling circle amplification on the insert DNA nano ballto extend a 3'-end of the template strand to generate the second partial template strand, the second partial template strand being used as the read1 strand sequencing template;
dephosphorylating the 3 '-end phosphorylated read1 strand sequencing primers, generating the read2 strand sequencing template by performing the multiple displacement amplification, and hybridizing the read2 strand sequencing template with the read2 strand sequencing primers;
hybridizing the read1 strand sequencing template with the read1 strand sequencing primers to obtaina DNA nano ball template ready to be sequenced, the DNA nano ball template being hybridized with the read1 strand sequencing primers and the read2 strand sequencing primers; and
simultaneously performing the read1 strand sequencing and the read2 strand sequencing using the read1 strand sequencing primers and the read2 strand sequencing primers, to obtain the sequences of the sense strand and the antisense strand of the insert DNA.

4. The method according to claim 1, comprising:
performing the first round of rolling circle amplification using a single-stranded circular DNA molecule of the insert DNA as the amplification template, to generate a DNA nano ball;
hybridizing the first partial template strand generated by the first round of rolling circle amplification with multiple-displacement-amplification primers;
performing the second round of rolling circle amplification on the insert DNA nano ball to extend a 3'-end of the template strand to generate the second partial template strand, the second partial template strand being used as the read1 strand sequencing template;
generating the read2 strand sequencing template by performing the multiple displacement amplification using the multiple-displacement-amplification primers, and hybridizing the read2 strand sequencing template with the read2 strand sequencing primers;
hybridizing the read1 strand sequencing template with the read1 strand sequencing primers to obtaina DNA nano ball template ready to be sequenced, the insert DNA nano ball template being hybridized with the read1 strand sequencing primers and the read2 strand sequencing primers; and
simultaneously performing the read1 strand sequencing and the read2 strand sequencing using the read1 strand sequencing primers and the read2 strand sequencing primers, to obtain the sequences of the sense strand and the antisense strand of theinsertDNA.

5. The method according to claim 1, comprising:
performing the first round of rolling circle amplification using a single-stranded circular DNA molecule of the insert DNA as the amplification template, to generate a DNA nano ball;
hybridizing the first partial template strand generated by the first round of rolling circle amplification with 3'-end phosphorylated read1 strand sequencing primers;
performing the second round of rolling circle amplification on the DNA nano ball to extend a 3'-end of the template strand, wherein in the second round of rolling circle amplification, a dUTP-containing mix is used first to extend the template strand to obtain a template strand having a part containing U bases, and a dNTP mix is used then to further extend the template strand to generate the second partial template strand;
hybridizing the second partial template strand with multiple-displacement-amplification primers, and performing the multiple displacement amplification to generate the read2 strand sequencing template;
hybridizing the read2 strand sequencing template with the read2 strand sequencing primers, and dephosphorylating the3'-end phosphorylated read1 strand sequencing primers to obtain a DNA nano ball template ready to be sequenced, the DNA nano ball template being hybridized with the read1 strand sequencing primers and the read2 strand sequencing primers; and
simultaneously performing the read1 strand sequencing and the read2 strand sequencing using the read1 strand sequencing primers and the read2 strand sequencing primers, to obtain the sequences of the sense strand and the antisense strand of the insert DNA.

6. The method according to any one of claims1to 5, wherein a copy number of the read1 strand sequencing template and a copy number of the read2 strand sequencing template are controlled by controlling time of the rolling circle amplification and time of the multiple displacement amplification.

7. The method according to claim 6, wherein the copy number of the read1 strand sequencing template and the copy number of the read2 strand sequencing template have a difference in folds, and sequencing bases are positioned by using a signal difference caused by the difference in folds during the sequencing.

8. The method according to any one of claims1to 5, wherein different specific antibodies with different fluorescence are added during the sequencing to specifically recognize different bases with blocking groups to acquire base signals.

9. The method according to claim 8, wherein the sequencing comprises:
firstly adding the bases with blocking groups,
adding specific antibodies with different fluorescence that are capable of specifically recognizing bases with blocking groups and acquiring signals,
eluting the antibodies to remove the signals, and subsequently removing the blocking groups, to enter a next round of cycle.

10. The method according to any one of claims1to 5, wherein the sequencing is performed on a MGISEQ-2000 sequencer.

## Patentansprüche

1. Verfahren zum synchronen Sequenzieren eines Sinn-Strangs und eines Antisinn-Strangs einer Insert-DNA, umfassend:
Durchführen einer ersten Runde einer Rolling-Circle-Amplifikation unter Verwendung eines zirkulären DNA-Moleküls der zu sequenzierenden Insert-DNA als Amplifikationsvorlage, um einen ersten partiellen Vorlagenstrang zu erzeugen;
Hybridisieren des ersten partiellen Vorlagenstrangs mit blockierten oder unblockierten Amplifikationsprimern, und Durchführen einer zweiten Runde einer Rolling-Circle-Amplifikation unter Verwendung des zirkulären DNA-Moleküls als Amplifikationsvorlage, um einen zweiten partiellen Vorlagenstrang zu erzeugen;
Auswählen eines partiellen Vorlagenstrangs des ersten partiellen Vorlagenstrangs und des zweiten partiellen Vorlagenstrangs als Read1-Strang-Sequenzierungsvorlage, und Erzeugen einer Read2-Strang-Sequenzierungsvorlage durch Durchführen einer Multiple-Displacement-Amplification unter Verwendung des anderen partiellen Vorlagenstrangs als Amplifikationsvorlage; und
Hybridisieren der Read1-Strang-Sequenzierungsvorlage mit Read1-Strang-Sequenzierungsprimern, Hybridisieren der Read2-Strang-Sequenzierungsvorlage mit Read2-Strang-Sequenzierungsprimern, und gleichzeitiges Durchführen einer Read1-Strang-Sequenzierung und einer Read2-Strang-Sequenzierung, um Sequenzen des Sinn-Strangs und des Antisinn-Strangs der Insert-DNA zu erhalten.

2. Verfahren nach Anspruch 1, wobei die blockierten Amplifikationsprimer 3'-End phosphorylierte Read1-Strang-Sequenzierungsprimer sind; und die unblockierten Amplifikationsprimer Multiple-Displacement-Amplificationsprimer sind.

3. Verfahren nach Anspruch 1, umfassend:
Durchführen der ersten Runde der Rolling-Circle-Amplifikation unter Verwendung eines einzelsträngigen zirkulären DNA-Moleküls der Insert-DNA als Amplifikationsvorlage, um einen Insert-DNA-Nanoball zu erzeugen;
Hybridisieren des ersten partiellen Vorlagenstrangs, der durch die erste Runde der Rolling-Circle-Amplification erzeugt wurde, mit den 3'-End phosphorylierten Read1-Strang-Sequenzierungsprimern;
Durchführen der zweiten Runde der Rolling-Circle-Amplification an dem Insert-DNA-Nanoball, um ein 3'-Ende des Vorlagenstrangs zu verlängern und den zweiten partiellen Vorlagenstrang zu erzeugen, wobei der zweite partielle Vorlagenstrang als Read1-Strang-Sequenzierungsvorlage verwendet wird;
Dephosphorylieren der 3'-Ende phosphorylierten Read1-Strang-Sequenzierungsprimer, Erzeugen der Read2-Strang-Sequenzierungsvorlage durch Durchführen der multiplen Displacement-Amplification, und Hybridisieren der Read2-Strang-Sequenzierungsvorlage mit den Read2-Strang-Sequenzierungsprimern;
Hybridisieren der Read1-Strang-Sequenzierungsvorlage mit den Read1-Strang-Sequenzierungsprimern, um eine DNA-Nanoball-Vorlage zu erhalten, die zur Sequenzierung bereit ist, wobei die DNA-Nanoball-Vorlage mit den Read1-Strang-Sequenzierungsprimern und den Read2-Strang-Sequenzierungsprimern hybridisiert wird; und
gleichzeitiges Durchführen einer Read1-Strang-Sequenzierung und einer Read2-Strang-Sequenzierung unter Verwendung der Read1-Strang-Sequenzierungsprimer und der Read2-Strang-Sequenzierungsprimer, um Sequenzen des Sinn-Strangs und des Antisinn-Strangs der Insert-DNA zu erhalten.

4. Verfahren nach Anspruch 1, umfassend:
Durchführen der ersten Runde der Rolling-Circle-Amplifikation unter Verwendung eines einzelsträngigen zirkulären DNA-Moleküls der Insert-DNA als Amplifikationsvorlage, um einen DNA-Nanoball zu erzeugen;
Hybridisieren des ersten partiellen Vorlagenstrangs, der durch die erste Runde der Rolling-Circle-Amplification erzeugt wurde, mit den Multiple-Displacement-Amplification-Primern;
Durchführen der zweiten Runde der Rolling-Circle-Amplification an dem Insert-DNA-Nanoball, um ein 3'-Ende des Vorlagenstrangs zu verlängern und den zweiten partiellen Vorlagenstrang zu erzeugen, wobei der zweite partielle Vorlagenstrang als Read1-Strang-Sequenzierungsvorlage verwendet wird;
Erzeugen der Read2-Strang-Sequenzierungsvorlage durch Durchführen der Multiple-Displacement Amplification unter Verwendung der Multiple-Displacement-Amplification-Primer, und Hybridisieren der Read2-Strang-Sequenzierungsvorlage mit den Read2-Strang-Sequenzierungsprimern;
Hybridisieren der Read1-Strang-Sequenzierungsvorlage mit den Read1-Strang-Sequenzierungsprimern, um eine DNA-Nanoball-Vorlage zu erhalten, die zur Sequenzierung bereit ist, wobei die Insert-DNA-Nanoball-Vorlage mit den Read1-Strang-Sequenzierungsprimern und den Read2-Strang-Sequenzierungsprimern hybridisiert wird; und
gleichzeitiges Durchführen einer Read1-Strang-Sequenzierung und einer Read2-Strang-Sequenzierung unter Verwendung der Read1-Strang-Sequenzierungsprimer und der Read2-Strang-Sequenzierungsprimer, um Sequenzen des Sinn-Strangs und des Antisinn-Strangs der Insert-DNA zu erhalten.

5. Verfahren nach Anspruch 1, umfassend:
Durchführen der ersten Runde der Rolling-Circle-Amplifikation unter Verwendung eines einzelsträngigen zirkulären DNA-Moleküls der Insert-DNA als Amplifikationsvorlage, um einen DNA-Nanoball zu erzeugen;
Hybridisieren des ersten partiellen Vorlagenstrangs, der durch die erste Runde der Rolling-Circle-Amplification erzeugt wurde, mit dem 3'-End phosphorylierten Read1-Strang-Sequenzierungsprimern;
Durchführen der zweiten Runde der Rolling-Circle-Amplification an der DNA-Nanoball, um ein 3'-Ende des Vorlagenstrangs zu verlängern, wobei in der zweiten Runde der Rolling-Circle-Amplification zuerst eine dUTP enthaltende Mischung zur Verlängerung des Vorlagenstrangs verwendet wird, um einen Vorlagenstrang mit einem U-Basen enthaltenden Teil zu erhalten, und dann eine dNTP-Mischung zur weiteren Verlängerung des Vorlagenstrangs verwendet wird, um den zweiten partiellen Vorlagenstrang zu erzeugen;
Hybridisieren des zweiten partiellen Vorlagenstrangs mit den Multiple-Displacement-Amplification-Primern, und Durchführen der Multiple-Displacement-Amplification, um die Read2-Strang-Sequenzierungsvorlage zu erzeugen;
Hybridisieren der Read2-Strang-Sequenzierungsvorlage mit den Read2-Strang-Sequenzierungsprimern, und Dephosphorylieren der 3'-Ende phosphorylierten Read1-Strang-Sequenzierungsprimer, um eine DNA-Nanoball-Vorlage zu erhalten, die zur Sequenzierung bereit ist, wobei die DNA-Nanoball-Vorlage mit den Read1-Strang-Sequenzierungsprimern und den Read2-Strang-Sequenzierungsprimern hybridisiert wird; und
gleichzeitiges Durchführen einer Read1-Strang-Sequenzierung und einer Read2-Strang-Sequenzierung unter Verwendung der Read1-Strang-Sequenzierungsprimer und der Read2-Strang-Sequenzierungsprimer, um Sequenzen des Sinn-Strangs und des Antisinn-Strangs der Insert-DNA zu erhalten.

6. Verfahren nach einem der Ansprüche 1 bis 5, wobei eine Kopienzahl der Read1-Strang-Sequenzierungsvorlage und eine Kopienzahl der Read2-Strang-Sequenzierungsvorlage durch Steuern der Zeitdauer der Rolling-Circle-Amplification und der Zeitdauer der Multiple-Displacement-Amplification gesteuert werden.

7. Verfahren nach Anspruch 6, wobei die Kopienzahl der Read1-Strang-Sequenzierungsvorlage und die Kopienzahl der Read1-Strang-Sequenzierungsvorlage einen fachen Unterschied aufweisen, und wobei Sequenzierungsbasen durch Verwenden eines Signalunterschieds positioniert werden, der durch den fachen Unterschied während der Sequenzierung verursacht wird.

8. Verfahren nach einem der Ansprüche 1 bis 5, wobei während der Sequenzierung unterschiedliche spezifische Antikörper mit unterschiedlicher Fluoreszenz hinzugefügt werden, um unterschiedliche Basen mit Blockierungsgruppen spezifisch zu erkennen und somit Basissignale zu erfassen.

9. Verfahren nach Anspruch 8, wobei die Sequenzierung umfasst:
Hinzufügen der Basen mit Blockierungsgruppen,
Hinzufügen der spezifischen Antikörper mit unterschiedlicher Fluoreszenz, die Basen mit Blockierungsgruppen spezifisch erkennen können, und Erfassen der Signale;
Eluieren der Antikörper, um die Signale zu entfernen, und anschließendes Entfernen der Blockierungsgruppen, um in eine nächste Zyklusrunde einzutreten.

10. Verfahren nach einem der Ansprüche 1 bis 5, wobei die Sequenzierung auf einem MGISEQ-2000-Sequenzierer durchgeführt wird.

## Revendications

1. Une méthode de séquençage simultané de la chaîne sens et de la chaîne anti-sens de l'ADN inséré, dans laquelle la méthode comprend les étapes suivantes :
Utiliser ladite molécule d'ADN circulaire de l'ADN inséré à séquencer comme matrice d'amplification pour une première amplification en boucle roulante afin de générer une première partie de chaîne matrice ;
Hybrider ladite première partie de chaîne matrice avec des amorces d'amplification bloquées ou non bloquées, et utiliser la molécule d'ADN circulaire comme matrice d'amplification pour une deuxième amplification en boucle roulante afin de générer une deuxième partie de chaîne matrice ;
Choisir l'une quelconque desdites première et deuxième parties de chaîne matrice comme matrice de séquençage de la chaîne read1, et générer une matrice de séquençage de la chaîne read2 en utilisant l'autre partie de chaîne matrice comme matrice d'amplification pour une amplification par substitution multiple ;
Hybrider ladite matrice de séquençage de la chaîne read1 avec une amorce de séquençage de la chaîne read1, hybrider la matrice de séquençage de ladite chaîne read2 avec une amorce de séquençage de la chaîne read2, et effectuer simultanément le séquençage de la chaîne read1 et le séquençage de la chaîne read2 afin d'obtenir la séquence de la chaîne sens et de ladite chaîne anti-sens de l'ADN inséré.

2. Le procédé selon la revendication 1, dans lequel les amorces d'amplification bloquées sont desdites amorces de séquençage de la chaîne read1 phosphorylées en 3' ; et lesdites amorces d'amplification non bloquées sont des amorces d'amplification par substitution multiple.

3. Le procédé selon la revendication 1, comprenant les étapes suivantes :
Utiliser la molécule d'ADN circulaire simple brin de l'ADN inséré comme matrice d'amplification pour la première amplification en boucle roulante afin de générer des nanosphères d'ADN inséré ;
Hybrider ladite première partie de chaîne matrice générée par la première amplification en boucle roulante avec des amorces de séquençage de la chaîne read1 phosphorylées en 3' ;
Effectuer ladite deuxième amplification en boucle roulante sur lesdites nanosphères d'ADN inséré pour allonger l'extrémité 3' de la chaîne matrice, afin de générer la deuxième partie de chaîne matrice, ladite deuxième partie de chaîne matrice est utilisée comme matrice de séquençage de la chaîne read1 ;
Dephosphoryler lesdites amorces de séquençage de la chaîne read1 phosphorylées en 3', et générer la matrice de séquençage de la chaîne read2 en effectuant une amplification par substitution multiple, puis hybrider ladite matrice de séquençage de la chaîne read2 avec lesdites amorces de séquençage de la chaîne read2 ;
Hybrider ladite matrice de séquençage de la chaîne read1 avec les amorces de séquençage de la chaîne read1 pour obtenir une matrice de nanosphères d'ADN prête pour le séquençage sur machine, sur laite matrice de nanosphères d'ADN sont hybridées lesdites amorces de séquençage de la chaîne read1 et lesdites amorces de séquençage de la chaîne read2 ; ainsi que
Utiliser lesdites amorces de séquençage de la chaîne read1 et lesdites amorces de séquençage de la chaîne read2 pour effectuer simultanément le séquençage de la chaîne read1 et le séquençage de la chaîne read2 afin d'obtenir ladite séquence de la chaîne sens et de la chaîne anti-sens de l'ADN inséré.

4. Le procédé selon la revendication 1, dans lequel comprend les étapes suivantes :
Utiliser la molécule d'ADN circulaire simple brin de l'ADN inséré comme matrice d'amplification pour ladite première amplification en boucle roulante afin de générer des nanosphères d'ADN ;
Hybrider ladite première partie de chaîne matrice générée par ladite première amplification en boucle roulante avec des amorces d'amplification par substitution multiple ;
Continuer ladite deuxième amplification en boucle roulante sur les nanosphères d'ADN inséré pour allonger l'extrémité 3' de la chaîne matrice et ainsi générer la deuxième partie de chaîne matrice, et ladite deuxième partie de chaîne matrice est utilisée comme matrice de séquençage de la chaîne read1 ;
Générer la matrice de séquençage de ladite chaîne read2 en utilisant les amorces d'amplification par substitution multiple pour effectuer une amplification par substitution multiple, puis hybrider ladite matrice de séquençage de la chaîne read2 avec lesdites amorces de séquençage de la chaîne read2 ;
Hybrider ladite matrice de séquençage de la chaîne read1 avec les amorces de séquençage de la chaîne read1 pour obtenir une matrice de nanosphères d'ADN inséré prête pour le séquençage sur machine, sur une matrice de nanosphères d'ADN inséré prête pour le séquençage sont hybridées lesdites amorces de séquençage de la chaîne read1 et lesdites amorces de séquençage de la chaîne read2 ;
Utiliser lesdites amorces de séquençage de la chaîne read1 et lesdites amorces de séquençage de la chaîne read2 pour effectuer simultanément le séquençage de la chaîne read1 et le séquençage de la chaîne read2 afin d'obtenir ladite séquence de la chaîne sens et de la chaîne anti-sens de l'ADN inséré.

5. Le procédé selon la revendication 1, il comprend les étapes suivantes :
Utiliser la molécule d'ADN circulaire simple brin de l'ADN inséré comme matrice d'amplification pour ladite première amplification en boucle roulante afin de générer des nanosphères d'ADN ;
Hybrider ladite première partie de chaîne matrice générée par la première amplification en boucle roulante avec des amorces de séquençage de la chaîne read1 phosphorylées en 3' ;
Continuer la deuxième amplification en boucle roulante sur les nanosphères d'ADN pour allonger l'extrémité 3' de ladite chaîne matrice. Dans cette deuxième amplification en boucle roulante, tout d'abord, allonger ladite chaîne matrice avec un mélange contenant du dUTP pour obtenir une chaîne matrice partiellement contenant des bases U, puis continuer d'allonger ladite chaîne matrice avec un mélange de dNTPs pour générer ladite deuxième partie de chaîne matrice ;
Hybrider ladite deuxième partie de chaîne matrice avec des amorces d'amplification par substitution multiple, puis effectuer une amplification par substitution multiple pour générer ladite matrice de séquençage de la chaîne read2 ;
Hybrider ladite matrice de séquençage de la chaîne read2 avec lesdites amorces de séquençage de la chaîne read2, et déphosphorylation des amorces de séquençage de ladite chaîne read1 phosphorylées en 3' pour obtenir une matrice de nanosphères d'ADN prête pour le séquençage sur machine, sur matrice de nanosphères d'ADN prête pour le séquençage sur machine sont hybridées lesdites amorces de séquençage de la chaîne read1 et lesdites amorces de séquençage de la chaîne read2 ;
Utiliser lesdites amorces de séquençage de la chaîne read1 et lesdites amorces de séquençage de la chaîne read2 pour effectuer simultanément le séquençage de la chaîne read1 et le séquençage de la chaîne read2 afin d'obtenir ladite séquence de la chaîne sens et de la chaîne anti-sens de l'ADN inséré.

6. Le procédé selon l'une quelconque des revendications 1 à 5, ledit nombre de copies de la matrice de séquençage de la chaîne read1 et ledit nombre de copies de la matrice de séquençage de la chaîne read2 sont contrôlés en régulant ledit temps d'amplification en boucle roulante et ledit temps d'amplification par substitution multiple.

7. Le procédé selon la revendication 6, dans lequel, il existe un rapport de multiplication entre ledit nombre de copies de la matrice de séquençage de la chaîne read1 et ledit nombre de copies de la matrice de séquençage de la chaîne read2. Lors du séquençage, utiliser la différence de signaux induite par ce rapport de multiplication pour localiser les bases séquencées.

8. Le procédé selon l'une quelconque des revendications 1 à 5, dans lequel, lors du séquençage, des anticorps spécifiques marqués avec différents fluorochromes sont ajoutés pour reconnaître spécifiquement les différentes bases porteuses de groupements bloquants, afin de collecter les signaux des bases.

9. Le procédé selon la revendication 8, le séquençage dans lequel, comprend les étapes suivantes :
Ajouter des bases porteuses de groupements bloquants ;
Ajouter des anti-corps spécifiques marqués avec différents fluorochromes capables de reconnaître spécifiquement les bases porteuses de groupements bloquants et collecter les signaux ;
Effectuer une élution des anticorps pour éliminer les signaux, puis éliminer les groupements bloquants pour passer à la prochaine boucle.

10. Le procédé selon l'une quelconque des revendications 1 à 5, dans lequel, le séquençage est effectué sur le séquenceur MGISEQ-2000.
